# EUROPEAN PATENT SPECIFICATION

(11) **EP 1 458 384 B1**
(45) Date of publication and mention of the grant of the patent: **26.07.2006**
(21) Application number: 02788262.0
(22) Date of filing: 17.12.2002
(51) Int. Cl.: A61K 31/4422, A61K 9/20

(54) **AMLOPIDINE BEZYLATE TABLETS WITH IMPROVED STABILITY**
AMLOPIDINBEZYLAT-TABLETTEN MIT VERBESSERTER STABILITÄT
COMPRIMES D'AMLOPIDINE BEZYLATE POSSEDANT UNE STABILITE AMELIOREE

(30) Priority: 17.12.2001 HU 0105345
(43) Date of publication of application: 22.09.2004
(73) Proprietor: EGIS GYOGYSZERGYAR RT., 1106 Budapest (HU)
(72) Inventor: FEKETE, Pál, H-1147 Budapest (HU); KIRALYNE IGNACZ, Mária, H-1161 Budapest (HU); TÖMPE, Péter, H-1116 Budapest (HU); GORA, Lászloné, H-2117 Isaszeg (HU); SZENTGROTI, Pálné, H-1071 Budapest (HU); LEVENTISZNE HUSZAR, Magdolna, H-1125 Budapest (HU); THUROCZI, Pálné, H-1165 Budapest (HU); LONKAINE MAGYAR, Olga, H-1119 Budapest (HU)
(74) Representative: Hirsch & Associés
(86) International application number: PCT/HU2002/000145
(87) International publication number: WO 2003/051364

(56) References cited:
- EP-A- 0 244 944
- WO-A-03/032954
- DE-A- 19 857 716

## Description

This invention relates to amlodipine bezylate tablets with improved stability of the active ingredient and reduced in weight containing microcrystalline cellulose, a lubricant and a disintegrating agent. The invention also relates to a process for the preparation of the said tablets.

It is known that amlodipine bezylate, [2-[(2-aminoethoxy)-methyl]-4-(2-chlorophenyl)-1,4-dihydro-6-methyl-3-ethyl-3,5-pyridinedicarboxylic acid 5-methyl ester bezylate] is one of the most important representatives of pharmaceutical ingredients possessing calcium antagonistic activity. In the therapeutical practice tablets containing amlodipine bezylate in amounts corresponding to 2.5 mg, 5 mg and 10 mg of free amlodipine base are used. The amounts of the amlodipine bezylate in the tablets are 3.472 mg, 6.944 mg and 13.888 mg, respectively. In spite of the relatively small active ingredient content the weights of the known tablets are surprisingly high (100 mg, 200 mg and 400 mg, respectively), that is the concentration of the active ingredient in the known compositions hardly exceeds 3 %.

According to the publication L'informatore Farmaceutico, 1994, the quantitative composition of the known amlodipine bezylate tablets is as follows:

| | |
|---|---|
| Norvasc® tablet, 10 mg: | |
| amlodipine bezylate | 13.889 mg |
| microcrystalline cellulose | 248.111 mg |
| calcium hydrophosphate (anhydrous) | 126.0 mg |
| sodium carboxymethyl starch | 8.0 mg |
| magnesium stearate | 4.0 mg |
| nominal weight of the tablet | 400.0 mg |

To a person skilled in the art of tablet making the composition suggests unambiguously a direct compression technique, as wet granulation binding agents soluble in water or in any other solvent are not present at all, and the microcrystalline cellulose and anhydrous calcium hydrophosphate are excipients typically applied in direct compression techniques.

The unusually small concentration of the active ingredient applied in the above composition must have been a consequence of the bad tabletting characteristics and poor stability of the amlodipine bezylate. During the direct compression technique that must have been used for the preparation of the known tablets, the active ingredient is admixed with the different tabletting excipients (fillers, dry binding agents, disintegrating agents, glidant agents) in powdered form, and then the powder mixture is tabletted. It is well known that direct tabletting excipients can only slightly improve the poor tabletting characteristics of the active ingredient. At the same time a wet granulation technique capable of resulting in a better improvement in compressibility could not have been applied due to the chemical instability of the active ingredient, since drying following the wet granulation would lead to an increased decomposition of the active ingredient, and granulation in an organic solvent is very expensive, especially when environmental protection requirements are properly respected.

The preparations being now on the market have several drawbacks. Due to the large amount of the excipients the tablet size is relatively large, and large tablets are difficult to swallow, especially for aged patients, who are in the first place involved in the ingestion of pharmaceutical preparations containing amlodipine. Besides, due to the high excipient content the costs of materials of the manufacturing procedure are relatively high, and in case of a given production line the output is rather low.

When reproducing the known amlodipine bezylate tablet we have found that the stability thereof under the stipulated accelerated stability examinations fails to comply with the requirements. Examinations namely have shown that upon storage for a period of 3 months at a temperature of 40°C under a relative humidity (RH) of 75 % the amount of the oxidation decomposition products in the tablets of the above-specified composition exceeded the amount of 0.5 % allowed by the United States Pharmacopoeia 24 (USP 24), in paragraph "Amlodipine Tablets".

The composition of the tested tablets was as follows:

| Component | 2.5 mg | 5 mg | 10 mg |
|---|---|---|---|
| amlodipine bezylate | 3.475 mg | 6.95 mg | 13.90 mg |
| cellulose, micro-crystalline PH 102 | 62.025 mg | 124.05 mg | 248.10 mg |
| calcium hydrogen phosphate (anhydrous) | 31.500 mg | 63.00 mg | 126.00 mg |
| sodium carboxymethyl starch | 2.000 mg | 4.00 mg | 8.00 mg |
| magnesium stearate | 1.000 mg | 2.00 mg | 4.00 mg |
| average weight | 100.00 mg | 200.00 mg | 400.00 mg |

As technique direct compression was applied, that is the above components were sifted, homogonized, and the homogenizate was compressed to tablets on a Manesty B3B type rotary tabletting machine.

The starting examinations of the tablets gave suitable results. During accelerated stability examinations lasting for 3 months, however, according to HPLC the amount of the oxidation decomposition products exceeded the allowed value of 0.5 %.

The contamination profile of the tablets containing 2.5 mg of amlodipine, that is the concentration of the contamination appearing at a given retention time determined by HPLC is as follows:

| | | 3 months | | 6 months | |
|---|---|---|---|---|---|
| t_{R} (min) | initial | 40°C 75%RH | 30°C 60%RH | 40°C 75%RH | 30°C 60%RH |
| 0.65 ' | < 0.1 % | 0.1 % | 0.1 % | 0.8 % | 0.01 % |
| 8.50 ' | < 0.1 % | 0.61 % | 0.37 % | 0.95 % | 0.3 % |
| 10.70 ' | < 0.1 % | 0.1 % | 0.1 % | 0.034 % | 0.08 % |
| < 0.1 % | < 0.1 % | < 0.1 % | < 0.1 % | 0.1 % | 0.1 % |

The contamination profile of the tablets containing 5 mg of amlodipine, according to HPLC is as follows:

| | | 3 months | | 6 months | |
|---|---|---|---|---|---|
| t_{R} (min) | initial | 40°C 75%RH | 30°C 60%RH | 40°C 75%RH | 30°C 60%RH |
| 8.2 ' | 0.24 % | < 0.1 % | < 0.1 % | 0.2 % | < 0.1 % |
| 8.5 ' | < 0.1 % | 0.56 % | 0.37 % | 0.84 % | 0.53 % |
| < 10.77 ' | 0.1 % | 0.1 % | 0.11 % | 0.14 % | 0.14 % |

The contamination profile of tablets containing 10 mg of amlodipine, according to HPLC is as follows

| | | 3 months | | 6 months | |
|---|---|---|---|---|---|
| t_{R} (min) | initial | 40°C 75%RH | 30°C 60%RH | 40°C 75%RH | 30°C 60%RH |
| 7.9 ' | 0.21 % | < 0.1 % | < 0.1 % | 0.2 % | < 0.1 % |
| 8.55 ' | < 0.1 % | 0.46 % | 0.28 % | 0.67 % | 0.41 % |
| < 0.1 % | 0.11 % | 0.11 % | < 0.1 % | < 0.1 % | < 0.1 % |
| 10.77 ' | < 0.1 % | < 0.1 % | < 0.11 % | 0.13 % | 0.14 % |

:

Thus, during accelerated stability examinations the amount of the oxidation product appearing at a retention time of about 8.5 min increased significantly and exceeded the allowable maximum amount of 0.5 %.

For the production of amlodipine bezylate tablets in a suitable quality there was a need therefore to provide a composition ensuring a suitable stability.

In order to provide a composition ensuring a suitable stability of the tablets containing amlodipine bezylate as active ingredient, tablets containing 10 mg of active ingredient were prepared with the aid of different direct tabletting fillers, and the compactibility characteristics of the said tablets were examined. The contamination content was measured before and after subjecting the tablets to a thermal load of 60°C for 4 days.

The compositions of the tablets and the results of the examinations are given in Table I below.

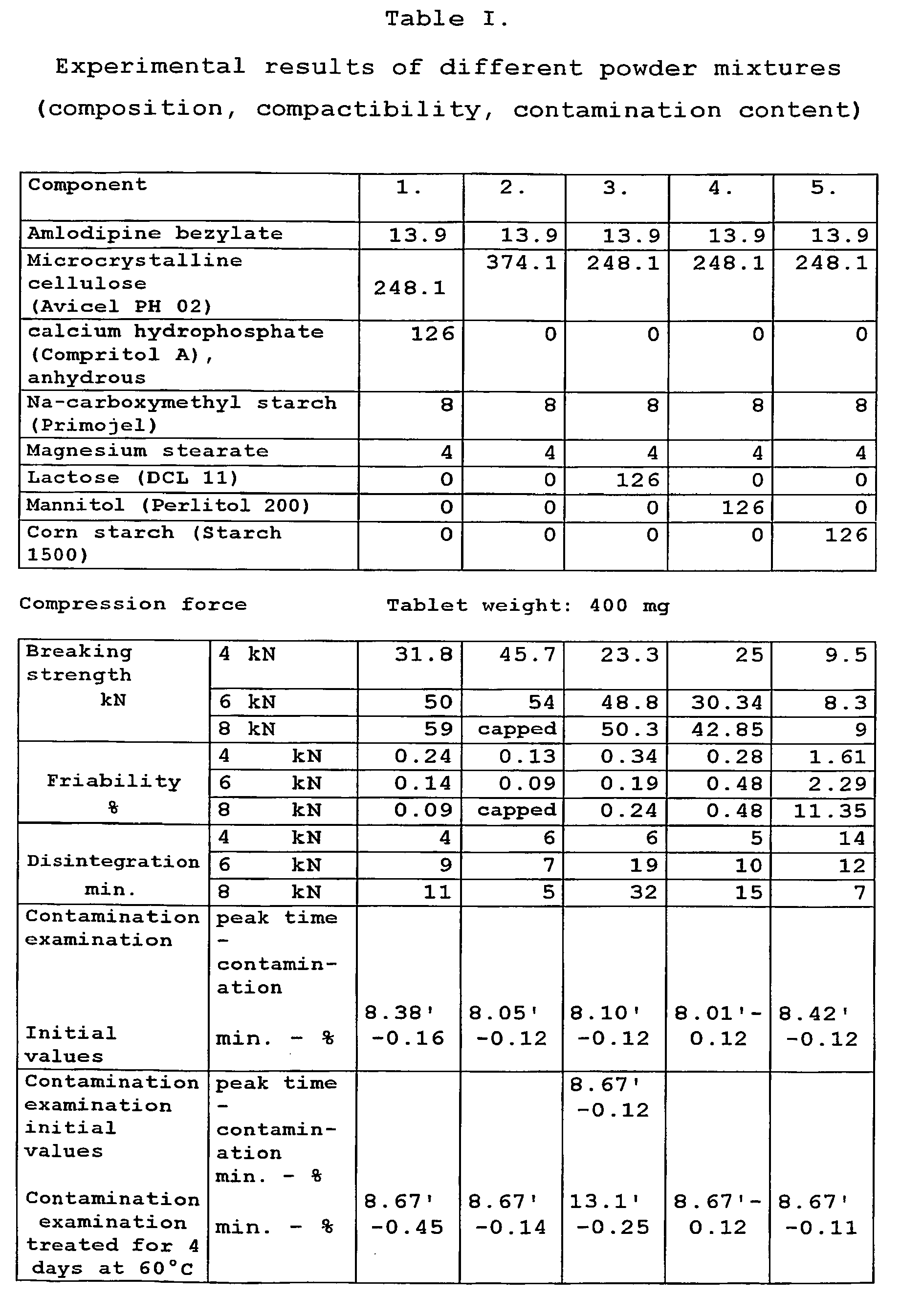

In the composition of the tablet calcium phosphate was substituted for specific fillers suitable for direct compression. As microcrystalline cellulose Avicel PH 102 from the firm FMC, as lactose Lactose DCL 11 from the firm DMV, as mannitol Perlitol 200 available from the firm Roquett, as corn starch Starch 1500 from the firm Colorcon was applied.

For the preparation of tablets the ingredients were homogenized in a gravity mixer and compressed into tablets on a Fette E XI type single punch tabletting machine under different pressing forces. The tablets were 10 mm in diameter and 400 mg in weight. The physical parameters of the tablets were measured by HPLC on the basis of the United States Pharmacopoeia 24 (USP 24), paragraph "Amlodipine Tablets". In order to assess the effect of the excipients on the chemical stability of the active ingredient the tablets were stored at a temperature of 60 °C for 4 days and the chemical analysis was repeated.

On the basis of the test results shown in Table I in case of the composition known from the state of the art the physical parameters of the tablets are satisfactory, the chemical stability thereof, however, is poor. Upon storing the tablets for 4 days at a temperature of 60 °C the amount of the oxidation products measured by HPLC has a peak at about 8 minutes and approaches the allowed upper limit of 0.5 %. In case of the other assessed direct compressible excipients the amount of the oxidation product did not increase as compared to the initial value, in case of lactose, however (experiment 3) a new, unknown contamination appeared in an amount of 0.25 % suggesting an incompatibility between the active ingredient and lactose. When applying Starch 1500, the corn starch proposed for direct compression, the mechanical properties of the tablets were weak with low breaking strength and very high friability. Tablets with the relatively most advantageous properties were obtained when using microcrystalline cellulose (experiment 2). In the latter case, namely, tablets showing the highest breaking strength and the shortest disintegration time were obtained by using a low compression force of 4 to 6 kN. The problem is that in case of the application of higher compression forces a laminar separation of the tablets occurred, so a further modification of the composition became necessary.

During the examination directed to adjusting a proper composition of the amlodipine bezylate tablets we have surprisingly found that lamination can be eliminated by reducing the amount of the microcrystalline cellulose possessing a very good compressibility per se, or by applying an amount of about 0.5 % by weight of colloidal silicon dioxide in the composition. The reduction of the amount of microcrystalline cellulose results in a considerable reduction in the weights of the tablets, too, which involves further advantages. First, a saving in the manufacturing costs can be achieved. Besides, as a consequence of the smaller tablet sizes, the swallowing of the tablets is facilitated, especially in case of tablets containing amlodipine bezylate corresponding to 10 mg of amlodipine base. The reduction in the amount of microcrystalline cellulose is, however, limited by the fact that the weight of the tablets containing the smallest amount of amlodipine bezylate corresponding to 2.5 mg amlodipine base may not be less than about 60 to 70 mg, preferably about 70 mg. Namely the lower limit of the tablet weight is determined by the tablet size physically manageable by the patients. This usually means a round tablet of 6 to 7 mm diameter, and the appropriate height of about 2.5 mm belonging to this diameter is optimally ensured by a tablet weight of about 60 to 70 mg.

According to an aspect of the present invention there are provided amlodipine bezylate tablets with improved stability of the active ingredient and reduced in weight containing microcrystalline cellulose, a lubricant and a disintegrating agent, which comprises 4-6 % by weight of amlodipine bezylate as active ingredient together with 87 to 94 % by weight of microcrystalline cellulose, 1-5 % by weight of a disintegrating agent, 0.5 to 1.5 % by weight of a lubricant and 0.2 to 1.0 % by weight of colloidal silicon dioxide.

The weight of the biggest tablets containing amlodipine bezylate in an amount corresponding to 10 mg of free amlodipine base amounts to 240 to 280 mg, preferably to about 280 mg.

According to our examinations it has been found that the most advantageous amlodipine bezylate tablets in respect of both the tablet manufacture or the physical parameters of the tablet and the chemical stability of the active ingredient are the ones containing about 5 % by weight of amlodipine bezylate, about 90 % by weight of microcrystalline cellulose, about 3 % by weight of a disintegrating agent (preferably sodium carboxymethyl starch), about 1% by weight of a lubricant (preferably magnesium stearate) and about 0.5% by weight of a glidant agent, preferably colloidal silicon dioxide. Instead of sodium carboxymethyl starch sodium or calcium carboxymethyl cellulose, crospovidone or low substituted hydroxypropyl cellulose (L-HPC) may also be used as a disintegrating agent for the tablets according to the invention. As lubricant instead of magnesium stearate calcium stearate may also be applied.

According to another aspect of the present invention there is provided the preparation of amlodipine bezylate tablets with improved stability of the active ingredient and reduced in weight, which comprises homogenizing 4-6 % by weight of amlodipine bezylate, 87 to 94 % by weight of microcrystalline cellulose, 1 to 5 % by weight of a disintegrating agent, 0.5 to 1.5 % by weight of a lubricant and 0.2 to 1.0 % by weight of colloidal silicon dioxide related to the total weight of the tablets by a powder mixing technique and compressing tablets from the thus-obtained powder mixture.

It can be seen that the preparation of the amlodipine bezylate tablets according to the invention is very simple. The ingredients are powdered and homogenized in the appropriate ratio and from the thus-obtained homogenizate tablets containing the desired amount of the active ingredient may be compressed on a tabletting machine of any type. According to our investigations the following compositions have proved to be very advantageous:

| Dose | 2.5 mg | 5 mg | 10 mg |
|---|---|---|---|
| Amlodipine bezylate | 3.475 m | 6.95 mg | 13.90 mg |
| Microcrystalline cellulose PH 102 | 63.400 m | 126.80 mg | 253.60 mg |
| Sodium carboxymethyl starch | 2.000 m | 4.00 mg | 8.00 mg |
| Magnesium stearate | 0.750 m | 1.50 mg | 3.00 mg |
| Colloidal silicon dioxide | 0.375 m | 0.75 mg | 1.50 mg |
| Average weight | 70.000 m | 140.00 mg | 280.00 mg |

The above-specified compositions are only of exemplary character, an alteration of the amounts of the individual components of about +/- 20 % does not lead to a lowering of the quality of the tablets.

In case of identical active ingredient contents the amlodipine bezylate tablets according to the invention are of considerably smaller weight than the known tablets. Thus an improved therapeutical applicability has been achieved, especially when tablets containing higher amounts of the active ingredient and bigger in size are prepared. Besides, the production of such tablets is more economical. The stability of the active ingredient in the tablets according to the invention complies even the strictest requirements of the pharmacopoeias.

Further details of the present invention are to be found in the following examples without limiting the scope of protection to the Examples.

### Example 1.

Preparation of tablets containing 10 mg of amlodipine
Batch size: 100,000 tablets (140 kg)
Technique: direct compression from a homogenizate
Composition of a batch:
A./ Premix:

| | |
|---|---|
| Amlodipine bezylate | 1.39 kg |
| Microcrystalline cellulose PH 102 FMC | 8.36 kg |
| Aerosil 200 | 0.15 kg |

B./Homogenizate

| | |
|---|---|
| Microcrystalline cellulose PH 102 FMC | 17.00 kg |
| Primojel (sodium carboxymethyl starch) | 0.80 kg |
| Magnesium stearate | 0.30 kg |

During the manufacturing procedure the ingredients measured for the premix are manually homogenized in a stainless steel vessel. Then about half of the amount of the microcrystalline cellulose is poured into a 100 litre barrel, the premix, the Primojel, the magnesium stearate and finally the residual amount of the microcrystalline cellulose are added to it and the components are homogenized by rotating the barrel with the aid of an appropriate apparatus. The homogenzate is compressed to tablets of 280 mg on a Manesty Betapress type tabletting machine using flat rimmed tabletting punches of 10 mm diameter. Each tablet contained 13.9 mg of amlodipine bezylate corresponding to 10 mg of amlodipine base.

The most important parameters of the tablets are as follows:

| Parameter | | | Results | Requirements |
|---|---|---|---|---|
| *outlook* | | | round, rimmed, | round, rimmed, |
| *colour* | | | almost white | white or almost white |
| *smell* | | | odourless | odourless |
| *size* | height | | 3.61- 3.71 mm | 3.5 mm ± 6 % (3.29 - 3.71 mm) |
| | diameter | | 10.05 - 10.08 mm | ~ 10 mm |
| *cutting surface* | | | almost white | white or almost white |
| *breaking strength* | | | 57 N | min. 50 N |
| *friability* | | | 0 % | max. 1 % |
| *average weight* | | | 0.2769 g | 280.0 mg ± 5 % 266.0 - 294.0 mg) |
| *weight uniformity* | min - max | | +2.71 % - 2.4 % | min. 18/20 average weight ± 5% |
| | | | | max. 2/20 average weight + 10 % |
| | RSD | | 1.27 % | max. 6 % |
| *identity* | HPLC | | identical | identical |
| *purity (HPLC)* | total contamination | | 0.09 % | max. 1 % |
| *oxidated derivative* | | | 8.8 ' - 0,07 % | max. 0.5 % |
| *unknown one by one* | | | 13.9 ' - 0.02 % | max. 0.1 % |
| *moisture content* | | KF | 3.59 % | max. 6 % |
| *disintegration time* | | | 1' | max. 15 ' |
| *dissolution of the active ingredient* | | | in 30 ' 97.7 % | in 30 ' min. 80 % (Q) |
| *active ingredient content* | | | 96.5 % | 13.90 mg ± 5 % (13.205- 14.595 mg) 10.00 mg ± 5 % (9.5-10.5 mg) |
| *content uniformity* | | min - max RSD | 96.8 - 100.7 % 1.33% | 85 - 115 % (related to the average of 10 tablets) |
| microbiological purity | | | | |
| Total aerobic microb. count (CFU /g) | | | suitable | max. 10³/g |
| mould + fungus(CFU g) | | | suitable | max. 10²/g |
| Escherichia coli (1g) | | | suitable | excluded |

The tablets were stored in a brown bottle closed with a polyethylene cap for 3 and 6 months at a temperature of 30°C under a relative humidity of 60 %, and at a temperature of 40°C under a relative humidity of 75 %, respectively.

After storage the most important parameters were as follows:

| | 3 months | | 6 months | |
|---|---|---|---|---|
| | 40°C 75%RH | 30°C 60%RH | 40°C 75%RH | 30°C 60%RH |
| colour | almost white | almost white | almost white | almost white |
| smell | odourless | odourless | odourless | odourless |
| undecomposed active ingredient | 97.4 % | 98.7 % | 97.9 % | 99.7 % |
| disintegration | 1' | 1' | 1' | 1' |
| strength | 54 N | 57 N | 52 N | 55 N |
| moisture content | 4.2 % | 3.96 % | 5.15 % | 4.77 % |
| friability | 0.0 % | 0.0 % | 0.0 % | 0.0 % |

**Contamination (HPLC ):**

| | | 3 months | | 6 months | |
|---|---|---|---|---|---|
| t_{R} (min) | initial | 40°C 75%RH | 30°C 60%RH | 40°C 75%RH | 30°C 60%RH |
| 3.6 ' | < 0.01 % | 0.07 % | 0,08 % | 0.03 % | 0.03 % |
| 6.3 ' | < 0.01 % | < 0.01 % | < 0.01 % | 0.09 % | 0.06 % |
| 8.8 ' | 0.07 % | 0.13 % | 0.13 % | 0.14 % | 0.14 % |
| 9.7 ' | < 0.01 % | < 0.01 % | < 0.01 % | 0.12 % | 0.12 % |
| 11.2 ' | < 0.01 % | 0.03 % | 0.04 % | < 0.01 % | < 0.01 % |
| 12.5 ' | < 0.01 % | < 0.01 % | < 0.01 % | 0.03 % | 0.02 % |
| 13.9 ' | 0.02 % | 0.03 % | 0.04 % | < 0.01 % | < 0.01 % |
| 15.5 ' | < 0.01 % | < 0.01 % | < 0.01 % | 0.03 % | 0.03 % |
| total | 0.09 % | 0.26 % | 0.29 % | 0.42 % | 0.40 % |

Dissolution of the active ingredient (%):
Dissolution apparatus: Ph.Eur. paddle, 50 rpm
Dissolution medium: 900 ml 0,1 N hydrogen chloride

| | initial | | | 40 °C / 75 %RH 3 months | | | 40 °C / 75 %RH 6 months | | |
|---|---|---|---|---|---|---|---|---|---|
| *time (min)* | *5* | *15* | *30* | *5* | *15* | *30* | *5* | *15* | *30* |
| average % | 94.8 | 97.5 | 97.7 | 98.8 | 98.5 | 98.7 | 97.2 | 99.5 | 99.4 |
| RSD % | 1.91 | 0.98 | 1.13 | 1.75 | 1.29 | 1.46 | 1.82 | 1.57 | 1.49 |

On the basis of the above experiments it can be established that both the quality and the stability of the amlodipine tablets containing 10 mg of active ingredient prepared according to the invention are fully satisfactory.

### Example 2.

### Preparation of tablets containing 5 mg of amlodipine

For the preparation of tablets containing 5 mg of amlodipine the homogenizate was prepared as described in Example 1. Then 14.0 kg of the homogenizate was measured and pressed to tablets weighting 140 mg on a Manesty Betapress type tabletting machine using flat rimmed tabletting punches of 8 mm diameter. Each tablet contained 6.95 mg of amlodipine bezylate corresponding to 5.0 mg of amlodipine base.

The most important parameters of the tablets are as follows:

| Parameter | | | Results | Requirements |
|---|---|---|---|---|
| *outlook* | | | round, rimmed | round, rimmed |
| *colour* | | | almost white | white or almost white |
| *smell* | | | odourless | odourless |
| *size* | height | | 2.47-2.52 mm | 2.45 mm ± 6 % (2.3-2.6 mm) |
| | diameter | | 8.02-8.09 mm | ~ 8 mm |
| *breaking surface* | | | almost white | white or almost white |
| *breaking strength* | | | 84 N (78-92) | min. 40 N |
| *average weight* | | | 140.1 mg | 140.0 mg ± 7.5 % (129.5-150.5 mg) |
| *weight uniformity* | min - max | | min.: - 1.78 %, 137.6 mg max.: +3.07 %, 144.4 mg | min. 18/20 averagrageight + 2/20 average weight + 15 % |
| *identity* | HPLC | | identical | identical |
| *purity (HPLC)* | total contamination | | 0.08% | max. 1 % |
| *oxidated derivative* | | | 8.7' - 0.08 % | max. 0.5 % |
| *unknown one by one* | | | 0 | max. 0.1 % |
| *moisture content* | | KF | 3.97 % | max. 6 % |
| *disintegration time* | | | 1 min. | max. 15 min. |
| *friability* | | | 0.09 % | min. 1% |
| *release of the active ingredient* | | | in 30 ' 99.0 % | in 30 min. 80 % (Q) |
| *release of the active ingredient* | | | 6.89 mg, 99.2 % | 6.95 mg ± 5% (6.602-7.2975) |
| *content uniformity* | | min - max RSD | 97.4 - 101.0 % 1.19 | 85 - 115 % (related to the average of 10 tablets) RSD: max. 6 % |
| Microbiological purity | | | | |
| Total aerobic microb. count (CFU /g) | | | suitable | max. 10³/g |
| mould + fungus (CFU /g) | | | suitable | max. 10²/g |
| Escherichia coli (1g) | | | suitable | excluded |

The tablets were stored in a brown bottle closed with a polyethylene cap for 3 and 6 months at a temperature of 30 °C under a relative humidity of 60 %, and at a temperature of 40°C under a relative humidity of 75 %, respectively.

After storage the most important parameters were as follows:

| | 3 months | | 6 months | |
|---|---|---|---|---|
| | 40°C/75%RH | 30°C/60%RH | 40°C/75%RH | 30°C/60%RH |
| colour | almost white | almost white | almost white | almost white |
| odour | odourless | odourless | odourless | odourless |
| undecomposed | 101.1 % | 99.5 % | 98.2 % | 98.0 % |
| active ingredient | 7.02 mg | 6.91 mg | 6.825 mg | 6.811 mg |
| disintegration | 1' | 1' | 1' | 1' |
| strength | 77 N (74-83) | 81 N (75-88) | 73 N (72-77 N) | 76 N (70-83 N) |
| moisture content | 4.85 % | 4.73 % | 5.28 % | 5.14 % |
| friability | 0.00 % | 0.00 % | 0.03 % | 0.06 % |

**Contamination (HPLC):**

| | | 3 months | | 6 months | |
|---|---|---|---|---|---|
| t_{R} min. | initial | 40 °C/75% RH | 30°C/60%RH | 40°C/75%RH | 30°C/60%RH |
| 2.4 ' | < 0.02 % | 0.08 % | 0.04 % | < 0.02 % | < 0.02 % |
| 3.6 ' | < 0.02 % | 0.02 % | 0.09 % | 0.03 % | 0.03 % |
| 6.3 ' | < 0.02 % | < 0.02 % | < 0.09 % | 0.03 % | < 0.02 % |
| 11.3 ' | < 0.02 % | < 0.02 % | 0.03 % | < 0.02 % | < 0.02 % |
| 13.9 ' | < 0.02 % | < 0.02 % | 0.04 % | < 0.02 % | < 0.02 % |
| total | | 0.15 % | 0.14 % | | |

Dissolution of the active ingredient (%):
Dissolution apparatus: Ph.Eur. paddle, 50 rpm
Dissolution medium: 900 ml 0,1 N hydrogen chloride

| | initial | | | 40 °C / 75 %RH 3 months | | | 40 °C / 75 %RH 6 months | | |
|---|---|---|---|---|---|---|---|---|---|
| *time (min)* | *5* | *15* | *30* | *5* | *15* | *30* | *5* | *15* | *30* |
| average % | 77.2 | 98.9 | 99.0 | 92.9 | 99.5 | 99.7 | 95.5 | 99.2 | 98.1 |
| RSD % | 14.36 | 2.14 | 1.33 | 1.66 | 2.27 | 2.59 | 1.69 | 2.03 | 1.39 |

On the basis of the above experiments it can be established that both the quality and the stability of the amlodipine tablets containing 5 mg of active ingredient prepared according to the invention are fully statisfactory.

### Example 3

Preparation of tablets containing 2.5 mg of amlodipine

For the preparation of tablets containing 2.5 mg of amlodipine half of the amount of the homogenizate prepared for the production of the tablets containing 5 mg of amlodipine according to Example 2 was used. From the homogenizate weighting 14.0 kg tablets weighting 70 mg were pressed on a Manesty Betapress type tabletting machine using flat rimmed tabletting punches of 6 mm diameter. Each tablet contained 3.475 mg of amlodipine bezylate corresponding to 2.5 mg of amlodipine base.

The most important parameters of the tablets are as follows:

| Parameter | | Results | Requirements |
|---|---|---|---|
| *outlook* | | round, rimmed | round, rimmed |
| *colour* | | almost white | white or almost white |
| *odour* | | odourless | odourless |
| *size* | height | 2.17-2.22 mm | 2.4 mm ± 6 % (2.26-2.54 mm) |
| | diameter | 6.00-6.01 mm | ~ 6 mm |
| *breaking surface* | | almost white | white or almost white |
| *breaking strength* | | 60 N (54-63) | min. 20 N |
| *average weight* | | 69.8 mg | 70.0 mg ± 10 % (63.0-77.0 mg) |
| *weight uniformity* | min - max | min.: - 0.86 %, 69.2 mg max.: +1.00 %, 70.5 mg | min. 18/20 average weight ± 10 % min. 2/20 average weight + 20 % |
| *identity* | HPLC | identical | identical |
| *purity (HPLC)* | total contamination | 0.11 % | max. 1 % |
| *oxidated derivative* | | 8.7' - 0.11 | max. 0.5 % |
| *unknown* | one by one | 0 | max. 0.1 % |
| *moisture content* | KF | 3.72 % | max. 6 % |
| *disintegration time* | | 1 min. | max. 15 min. |
| *friability* | | 0.0 % | min. 1% |
| *release of the active ingredient* | | 98.8 % in 30' | min. 80 % in 30 (Q) |
| *active ingredient content* | | 3.435 mg, 98,86 % | 3.475 mg ± 5% (3.301-3.649) |
| *content uniformity* | min - max RSD | 99.0 - 102.2 % 0.95 | 85 - 115 % (10 related to the average of the tablet) RSD: max. 6 % |
| Microbiological purity Total aerobic microb. count (CFU /g) mould + fungus (CFU /g) Escherichia coli (1g) | | suitable | max. 10³/g |
| | | suitable | max. 10²/g |
| | | suitable | excluded |

The tablets were stored in a brown bottle closed with a polyethylene cap for 3 and 6 months at a temperature of 30 °C under a relative humidity of 60 % and at a temperature of 40°C under a relative humidity of 75 %, respectively.

After storage the most important parameters were as follows:

| | 3 months | | 6 months | |
|---|---|---|---|---|
| | 40°C/75%RH | 30°C/60%RH | 40°C/75%RH | 30°C/60%RH |
| colour | almost white | almost white | almost white | almost white |
| smell | odourless | odourless | odourless | odourless |
| undecomposed active ingredient | 99.9 % 3.472 mg | 100.2 % 3.482 mg | 97.8 % 3.398 mg | 98.2 % 3.412 mg |
| disintegration | 1' | 1' | 1' | 1' |
| strength | 55 N (51-60) | 57 N (54-61) | 53 N (51-56 N) | 55 N m(/U57) N |
| moisture content | 4.42 % | 4.49 % | 5.13 % | 4.95 % |
| friability | 0.00 % | 0.00 % | 0.00 % | 0.08 % |

**Contamination (HPLC ):**

| | | 3 months | | 6 months | |
|---|---|---|---|---|---|
| t_{R} (min) | initial | 40°C 75%RH | 30°C 60%RH | 40°C 75%RH | 30°C 60%RH |
| 3.6 ' | < 0.01 % | 0.02 % | < 0.01 % | 0.03 % | 0.04 % |
| 8.9 ' | 0.11 | 0.13 % | 0.14 % | 0.14 | 0.12 |
| 14.2 ' | < 0.01 % | < 0.01 % | 0.05 % | 0.03 % | 0.06% |
| total | 0.11 | 0.15 % | 0.14 % | 0.20 | 0.22 |

Dissolution of the active ingredient (%):
Dissolution apparatus: Ph.Eur. paddle, 50 rpm
Dissolution medium: 900 ml 0,1 N hydrogen chloride

| | initial | | | 40 °C / 75 %RH 3 months | | | 40 °C / 75 %RH 6 months | | |
|---|---|---|---|---|---|---|---|---|---|
| time (min) | *5* | *15* | *30* | *5* | *5* | *30* | *5* | *15* | *30* |
| average % | 93,9 | 99,1 | 98,8 | 93,9 | 99,9 | 100,2 | 96,8 | 98,5 | 99,7 |
| RSD % | 2,37 | 0,38 | 0,60 | 4,36 | 1,42 | 1,04 | 1,28 | 1,42 | 1,32 |

On the basis of the above experiments it can be established that both the quality and the stability of the amlodipine tablets containing 2.5 mg of active ingredient prepared according to the invention are fully satisfactory.

## Claims

1. Amlodipine bezylate tablets with improved stability of the active ingredient and reduced in weight containing microcrystalline cellulose, a lubricant and a disintegrating agent, which comprises 4-6 % by weight of amlodipine bezylate as active ingredient together with 87 to 94 % by weight of microcrystalline cellulose, 1-5 % by weight of a disintegrating agent, 0.5 to 1.5 % by weight of a lubricant and 0.2 to 1.0 % by weight of colloidal silicon dioxide.

2. Tablets as claimed in claim 1, wherein the tablets weight 60 to 80 mg and have an active ingredient content corresponding to 2.5 mg of amlodipine base.

3. Tablets as claimed in claim 1, wherein the tablets weight 120 to 160 mg and have an active ingredient content corresponding to 5 mg of amlodipine base.

4. Tablets as claimed in claim 1, wherein the tablets weight 240 to 320 mg and have an active ingredient content corresponding to 10 mg of amlodipine base.

5. A process for the preparation of amlodipine bezylate tablets as claimed in claim 1, which comprises homogenizing 4-6 % by weight of amlodipine bezylate, 87 to 94 % by weight of microcrystalline cellulose, 1 to 5 % by weight of a disintegrating agent, 0.5 to 1.5 % by weight of a lubricant and 0.2 to 1.0 % by weight of colloidal silicon dioxide related to the total weight of the tablets by a powder mixing technique and compressing tablets from the thus-obtained powder mixture.

6. A process as claimed in claim 5, which comprises compressing from the powder mixture tablets containing amlodipine bezylate in an amount corresponding to 2.5 mg, 5 mg or 10 mg of amlodipine base.

## Patentansprüche

1. Amlodipin-bezylat-Tabletten mit verbesserter Stabilität des Wirkstoffs und verringertem Gewicht, mikrokristalline Cellulose, ein Gleitmittel und ein Sprengmittel enthaltend, aufweisend 4 - 6 Gew.% Amlodipin-bezylat als Wirkstoff zusammen mit 87 - 94 Gew.% mikrokristalliner Cellulose, 1 - 5 Gew.% eines Sprengmittels, 0,5 - 1,5 Gew.% eines Gleitmittels und 0,2 - 1,0 Gew.% kolloidalem Siliciumdioxid.

2. Tabletten wie in Anspruch 1 beansprucht, wobei die Tabletten 60 - 80 mg wiegen und einen Wirkstoff-Gehalt haben, der 2,5 mg Amlodipin-Base entspricht.

3. Tabletten wie in Anspruch 1 beansprucht, wobei die Tabletten 120 - 160 mg wiegen und einen Wirkstoffgehalt haben, der 5 mg Amlodipin-Base entspricht.

4. Tabletten wie in Anspruch 1 beansprucht, wobei die Tabletten 240 - 320 mg wiegen und einen Wirkstoff-Gehalt haben, der 10 mg Amlodipin-Base entspricht.

5. Verfahren zur Herstellung von Amlodipin-bezylat-Tabletten wie in Anspruch 1 beansprucht, aufweisend ein Homogenisieren von 4 - 6 Gew.% Amlodipin-bezylat, 87 - 94 Gew.% mikrokristalliner Cellulose, 1 - 5 Gew.% eines Sprengmittels, 0,5- 1,5 Gew.% eines Gleitmittels und 0,2 - 1,0 Gew.% kolloidalem Siliciumdioxid, bezogen auf das Gesamtgewicht der Tabletten, mittels einer Pulvermischtechnik, und Pressen von Tabletten aus dem so erhaltenen Pulvergemisch.

6. Verfahren wie in Anspruch 5 beansprucht, aufweisend das Pressen von Tabletten, die Amlodipin-bezylat in einer 2,5 mg, 5 mg oder 10 mg Amlodipin-Base entsprechenden Menge enthalten, aus dem Pulvergemisch.

## Revendications

1. Comprimés de bézylate d'amlodipine, de stabilité de l'ingrédient actif améliorée et de poids diminué, contenant de la cellulose microcristalline, un lubrifiant et un agent désintégrant, qui comprend 4 à 6 % en poids de bézylate d'amlodipine en tant qu'ingrédient actif, associé avec de 87 à 94 % en poids de cellulose microcristalline, 1 à 5 % en poids d'un agent désintégrant, 0,5 à 1,5 % en poids d'un lubrifiant et de 0,2 à 1,0 % en poids de dioxyde de silicium colloïdal.

2. Comprimés selon la revendication 1, dans lesquels le poids des comprimés est de 60 à 80 mg et présentent une teneur en ingrédient actif correspondant à 2,5 mg de amlodipine base.

3. Comprimés selon la revendication 1, dans lesquels le poids des comprimés est de 120 à 160 mg et présentent une teneur en ingrédient actif correspondant à 5 mg d'amlodipine base.

4. Comprimés selon la revendication 1, dans lesquels le poids des comprimés est de 240 à 320 mg et présentent une teneur en ingrédient actif correspondant à 10 mg d'amlodipine base.

5. Un procédé de préparation de comprimés de bézylate d'amlodipine selon la revendication 1, qui comprend l'homogénéisation de 4 à 6 % en poids de bézylate d'amlodipine, de 87 à 94 % en poids de cellulose microcristalline, de 1 à 5 % en poids d'un agent désintégrant, de 0,5 à 1,5 % en poids d'un lubrifiant et de 0,2 à 1,0 % en poids de dioxyde de silicium colloïdal, exprimé par rapport au poids total des comprimés par une technique de malaxage de poudre et compression des comprimés à partir du mélange de poudre ainsi obtenu.

6. Un procédé selon la revendication 5, qui comprend une compression à partir des mélanges de poudre, de comprimés contenant du bézylate d'amlodipine à raison de 2,5 mg, 5 mg ou 10 mg d'amlodipine base.
